Europäisches Patentamt

European Patent Office

Office européen des brevets

(19)

(11) Veröffentlichungsnummer: **0 243 649**
**B1**

(12) # EUROPÄISCHE PATENTSCHRIFT

(45) Veröffentlichungstag der Patentschrift:
**12.09.90**

(51) Int. Cl.⁵: **A61K 7/06**

(21) Anmeldenummer: **87103963.2**

(22) Anmeldetag: **18.03.87**

(54) Mittel zur Wachstumsförderung der Haare.

(30) Priorität: **29.04.86 DE 3614448**

(43) Veröffentlichungstag der Anmeldung:
**04.11.87 Patentblatt 87/45**

(45) Bekanntmachung des Hinweises auf die Patenterteilung:
**12.09.90 Patentblatt 90/37**

(84) Benannte Vertragsstaaten:
**BE CH DE FR GB LI NL**

(56) Entgegenhaltungen:
**DE-A- 3 301 158**
**FR-A- 2 070 045**

(73) Patentinhaber: **Schnell, Guido, Rübackerstrasse 383,
CH-4244 Röschenz(CH)**

(72) Erfinder: **Schnell, Guido, Rübackerstrasse 383,
CH-4244 Röschenz(CH)**

(74) Vertreter: **Haft, Berngruber, Czybulka,
Postfach 14 02 46 Hans-Sachs-Strasse 5,
D-8000 München 5(DE)**

## Beschreibung

Die Erfindung bezieht sich auf ein Mittel zur Förderung des Wachstums und zur Erhaltung der Haare, insbesondere der menschlichen Kopfhaare.

Es sind schon große Anstrengungen unternommen worden, um ein Mittel zu finden, mit dem die menschlichen Kopfhaare wenigstens erhalten werden können, damit dem vor allem altersbedingten Haarausfall Einhalt geboten werden kann. So wurde beispielsweise vorgeschlagen, eine Mischung von Hühnereiweiß und Essigsäure (DE-PS 23 4l 029), ein Aminosäuregemisch (DE-PS 3l l8 882), Weizenkeim- oder Sojaöl (DE-OS 3l 23 943), Brennesselkonzentrat (DE-OS 3l 40 l6l), Beifuß vermischt mit Petersilie und vergorenen schwarzen Trauben (DE-PS 29 4l 748), bestimmte Vitamine (DE-PS 3l 43 624), konzentrierter Johannisbeersaft (DE-AS 22 l5 832) und ein Gemisch aus Fruchtkörnern von Ständerpilzen und Insektenlarven (DE-PS 32 42 446) als Haarwuchsmittel zu verwenden. Eine wirksame Eindämmung des Haarausfalls konnte mit diesen Mitteln allerdings nicht erzielt werden.

Der vorliegenden Erfindung liegt daher die Aufgabe zugrunde, ein Mittel zur Förderung und Erhaltung des Haarwuchses zu finden, welches eine außerordentlich gute Wirkung insbesondere am menschlichen Kopfhaar entfaltet.

Dies wird durch ein Mittel erreicht, welches folgende Bestandteile enthält: Sesamöl, Kha, Rahungthet-Kerne, Noinalod-Kerne, Galle des Schlammfischs (mud fish) und Ruß.

Vorteilhaft ist es, wenn die einzelnen Bestandteile in den im Anspruch 2 angegebenen Anteilen vorliegen.

Weiterhin ist es vorteilhaft, wenn das Mittel außerdem Phakchipa enthält, dessen bevorzugter Gehaltsbereich im Anspruch 4 wiedergegeben ist.

Im einzelnen ist zu den Bestandteilen des erfindungsgemäßen Mittels folgendes festzustellen:

Sesamöl ist bekanntlich ein aus Sesamkernen gepresstes Öl. Die wissenschaftliche Bezeichnung lautet "Sesamum orientale L". Wenn Sesamöl in die Haut eingerieben wird, macht es die Haut weich.

Kha ist eine Pflanze, die unter der Erdoberfläche wächst. Kha ist eine Pflanze der Gruppe Khing. Die wissenschaftliche Bezeichnung lautet "Alpinia galanga s.w.", wobei Kha zur Gruppe der Zingiberaceae gehört. Kha ist auch wirksam bei Hautkrankheiten.

Rahungthet-Kerne, die auch als Lahung- oder Rahung-Kerne bezeichnet werden, besitzen die wissenschaftliche Bezeichnung "Ricinus communis L.". Die Kerne weisen eine glatte Oberfläche und Streifen auf. Sie enthalten viel Öl, welches insbesondere als Abführmittel verwendet wird.

Noinalod-Kerne stellen Kerne der Pflanze Noina dar, die in Thailand weit verbreitet ist. Der wissenschaftliche Name dieser Pflanze lautet "Anona squamosa L.". Sie stammt aus der Gruppe der Anonaceae. Die englische Bezeichnung lautet "Custard apple tree" oder "Shugar apple tree". Die Kerne sind schwarz und klein. Sie werden bei Hauterkrankungen verwendet.

Phakchipa ist eine Pflanze, die zur gleichen Gruppe wie Phakchi gehört. Die Phakchipa-Pflanze weist gezackte und feine Blätter auf. Sie besitzt ein weiches Holz und trägt Früchte, die sehr klein sind, ähnlich den Früchten von Phakchi. Die wissenschaftliche Bezeichnung lautet "Petroselinum crispum L.". Phakchipa wird der Gruppe der Umbelliferae zugerechnet.

Beim Schlammfisch (mud fish) handelt es sich um eine im Schlamm oder schlammigen Gewässern lebende Fischsorte. Die Galle dieses Fisches wird nach dem Zerkleinern mit Sesamöl gemischt und zugegeben.

Als Ruß kann erfindungsgemäß jeder Kohlenstoff in feinstverteilter Form verwendet werden. Besonders feiner Ruß wird durch Verbrennung von Kolophonium, Naphthalin und Teerölen erhalten.

Die Herstellung des erfindungsgemäßen Mittels erfolgt dadurch, das Kha, die Rahungthet-Kerne, Noinalod-Kerne sowie gegebenenfalls Phakchipa fein zerhackt und dann durchgemischt werden, worauf Sesamöl, vermischt mit Schlammfisch-Galle und Ruß zugegeben wird und anschließend das Ganze innig vermischt wird.

### Beispiel

Es wurde ein Mittel folgender Zusammensetzung hergestellt:

Sesamöl 50 g
Schlammfisch-Galle 45 g
Phakchipa 10 g
Kha 20 g
Rahungthet-Kerne 15 g
Noinalod-Kerne 25 g
Ruß 2 g

Die Schlammfisch-Galle und das Sesamöl wurden vermischt. Das Phakchipa, Kha sowie die Rahungthet-Kerne und Noinalod-Kerne wurden einzeln geröstet, fein zerhackt, durchmischt und mit dem Sesamöl-Schlammfisch-Galle-Gemisch und dem Ruß versetzt, worauf das Ganze unter Bildung einer schwarzen Creme innig vermischt wurde.

Das Mittel wurde vom Erfinder (männlich, 35 Jahre) ausprobiert, der selbst unter sehr starkem Haarausfall litt. Er hat die schwarze Creme jeden zweiten Tag drei Wochen lang in die Kopfhaut einmassiert. Etwa 2 Wochen nach Beginn der Behandlung tauchte der erste schwache Haarwuchs auf der Glatze auf. Nach ca. 8 Wochen hatten die nachgewachsenen Haare eine Länge von 2 bis 3 mm. Weiterhin wurde festgestellt, daß zumindest während einer Dauer von 7 Monaten nach Beendigung der Kur kein Haarausfall mehr erfolgte.

Nach diesem deutlich sichtbaren Erfolg wurde das Mittel an vier weiteren Personen getestet, die unter Haarausfall litten. Nach vierwöchiger Behandlung wurde festgestellt, daß bei allen vier Versuchspersonen kein Haarausfall mehr auftrat und zudem auf ihren Glatzen wieder Haare wuchsen .

**Patentansprüche**

1. Mittel zur Förderung des Wachstums und zur Erhaltung der Haare, insbesondere der menschlichen Kopfhaare, enthaltend Sesamöl (Sesam orientale), dadurch gekennzeichnet, daß es als weitere Bestandteile geröstetes, zerkleinertes Kha (Alpinia galanga), geröstete, zerkleinerte Rahungthet-Kerne (aicinus communis), geröstete, zerkleinerte Noinalod-Kerne (Anona squamosa), zerkleinerte Schlammfisch-Galle und Ruß enthält.

2. Mittel nach Anspruch 1, dadurch gekennzeichnet, daß Bestandteile in folgenden Anteilen vorliegen:

Sesamöl (Sesam orientale) mindestens 20, vorzugsweise mindestens 40 Gew. –%
xha (Alpinia galanga) 5–35, vorzugsweise 15–25 Gew. -%
Rahungthet-Kerne (aicinus communis) 5–30, vorzugsweise 10–20 Gew. -%
Noinalod-Kerne (Anona squamosa) 5–50, vorzugsweise 15–35 Gew. -%
Schlammfisch-Galle 5–60, vorzugsweise 40–50 Gew. % Ruß 1–5, vorzugsweise 2–4 Gew.-%.

3. Mittel nach Anspruch 1, dadurch gekennzeichnet, daß es zusätzlich Phakchipa (Petroselinum crispum L) enthält.

4. Mittel nach Anspruch 3, dadurch gekennzeichnet, daß Phakchipa (Petroselinum crispum L) in einem Anteil von 1–30, vorzugsweise 5–15 Gew. -% vorliegt.

5. Verfahren zur Herstellung des Mittels nach einem der vorstehenden Ansprüche, dadurch gekennzeichnet, daß Kha (Alpinia galanga), die Rahungthet-Kerne (Ricinus communis), Noinalod-Kerne (Anona squamosa) sowie ggfs. Phakchipa (Petroselinum crispum L) geröstet, anschließend zerhackt und durchgemischt werden, worauf das Sesamöl (Sesam orientale), vermischt mit der Schlammfisch-Galle, und Ruß zugegeben und das Ganze vermischt wird.

**Claims**

1. An agent for promoting the growth and preservation of hair in particular human hair of the head, containing sesame oil (Sesamum orientale), characterized in that its further ingredients are roasted crushed kha (Alpinia galanga), roasted crushed rahungthet seeds (Ricinus communis), roasted crushed custard apple seeds (Annona squamosa), crushed mudfish gall and soot.

2. The agent of claim 1, characterized in that the ingredients are present in the following proportions:

sesame oil (Sesamum orientale) at least 20 wt.% preferably at least 40 wt.%,
kha (Alpinia galanga) 5 to 35 wt.%, preferably 15 to 25 wt.%,
rahungthet seeds (Ricinus communis) 5 to 30 wt.%, preferably 10 to 20 wt.%,
custard apple seeds (Annona squamosa) 5 to 50 wt.%, preferably 15 to 35% wt.%,
mudfish gall 5 to 60 wt.%, preferably 40 to 50 wt.%,
soot 1 to 5 wt.%, preferably 2 to 4 wt.%

3. The agent of claim 1, characterized in that it additionally contains phakchipa (Petroselinum crispum L).

4. The agent of claim 3, characterized in that phakchipa (Petroselinum crispum L) is present in a proportion of 1 to 30 wt.%, preferably 5 to 15 wt.%.

5. A method for producing the agent of any of the above claims, characterized by the steps of roasting kha (Alpinia galanga), rahungthet seeds (Ricinus communis), custard apple seeds (Annona squamosa) and optionally phakchipa (Petroselinum crispum L), thereafter crushing and mixing these substances, adding the sesame oil (Sesamum orientale) mixed with the mudfish gall, and soot, and mixing all substances together.

**Revendications**

1. Agent d'intensification de la pousse et de maintien des cheveux, notamment des cheveux humains, comprenant de l'huile de sésame (Sesam orientale), caractérisé en ce qu'il contient comme autres composants du kha (Alpinia galanga) grillé et broyé, des graines de rahunghet (Ricinus communis) broyées, des graines de noinalod (Anona squamosa) grillées et broyées, de la bile de poisson de vase et de la calamine.

2. Agent selon la revendication 1, caractérisé en ce que les composants sont compris dans les rapports suivants:

Huile de sésame (Sesam orientale), au moins 20 % en poids, de préférence 40 % en poids,
Kha (Alpinia galanga) 5 à 35 % en poids, de préférence 15 à 25 % en poids,
Graines de rahunghet (Ricinus communis), 5 à 30 % en poids, de préférence 10 à 20 % en poids,
Graines de noinalod (Anona squamosa), 5 à 50 % en poids, de préférence 15 à 35 % en poids,
Bile de poisson de vase, 5 à 60 % en poids, de préférence 40 à 50 % en poids,
Calamine, 1 à 5 % en poids, de préférence 2 à 4 % en poids.

3. Agent selon la revendication 1, caractérisé en ce qu'il contient en outre du phakchipa (Petroselinum crispum L).

4. Agent selon la revendication 3, caractérisé en ce que le phakchipa (Petroselinum crispum L) se trouve dans un rapport de 1 à 30 % en poids, de préférence de 5 à 15 % en poids.

5. Procédé de fabrication de l'agent selon l'une des revendications précédentes, caractérisé en ce que le kha (Alpinia galanga), les graines de rahunghet (Ricinus communis), les graines de noinalod (Anona squamosa) ainsi que le cas échéant le phakchipa (Petroselinum crispum L) sont grillés, ensuite hachés et mélangés, l'huile de sésame (Sesam orientale) étant mélangée avec la bile de poisson de vase, la calamine y étant ajoutée et le tout étant mélangé.